# EUROPEAN PATENT APPLICATION

(11) **EP 4 806 906 A1**
(43) Date of publication of application: **16.09.2026**
(21) Application number: 26164700.2
(22) Date of filing: 13.03.2026
(51) Int. Cl.: C07K 14/005

(54) **VIRUS-LIKE STRUCTURED NANO-CONNECTOR AND USE THEREOF**

(30) Priority: 13.03.2025 CN 202510296492
(71) Applicant: Eye Hospital of Shandong First Medical University (Shandong Eye Hospital), Jinan City, Shandong Province 250021 (CN)
(72) Inventor: Gao, Hua, Jinan City, Shandong Province, 250021 (CN); Li, Wenlong, Jinan City, Shandong Province, 250021 (CN); Bao, Qingdong, Jinan City, Shandong Province, 250021 (CN); Xie, Lixin, Jinan City, Shandong Province, 250021 (CN)
(74) Representative: karo IP

(57) **Abstract**

A virus-like structured nano-connector, it is based on a hepatitis B virus core protein uniprot P03146 with a C-terminal nucleic acid-binding domain of positions 145-183 (preferably 150-183) being removed, and its spike region and/or N-terminal are respectively inserted with CBP as shown in SEQ ID NO. 01. The present disclosure is constructed based on a collagen-binding polypeptide (CBP) designed by bionic decorin, it is intended to mimic interaction between the native decorin and collagen fibers, enabling efficient penetration across a corneal epithelial barrier and repairing corneal stroma via a non-photocrosslinking approach, thus overcoming limitations associated with the existing clinical therapies.

## Description

### RELATED APPLICATIONS

This application claims priority to Chinese patent application number 202510296492.1, filed on March 13, 2025. Chinese patent application number 202510296492.1 is incorporated herein by reference.

### FIELD OF THE DISCLOSURE

The present disclosure relates to the technical field of pharmaceutical carriers and ophthalmic therapeutic drugs, and specifically relates to a virus-like structured nano-connector and the use thereof.

### BACKGROUND OF THE DISCLOSURE

Corneal ectatic diseases are characterized by corneal weakness and irregular remodeling of corneal structure, with specific manifestations including corneal thinning, protrusion, and irregular astigmatism. Keratoconus is a bilateral, progressive and non-inflammatory corneal ectatic disease. This disease leads to increased paracentral corneal steepness and expanded range of corneal thinning, which in turn causes persistent progression of myopia, irregular astigmatism and visual loss, thus forming corneal scarring in an advanced stage. As the disease progresses, visual impairment usually begins in adolescence and continues to progress significantly until around the age of 35, which significantly reduces a life quality of the patient. In daily life, simple activities such as driving, recognizing a face of people at a distance or reading road signs may become difficult, therefore, effective technologies are in urgent need for immediate treatment of keratoconus patients. Currently, clinical treatments for the keratoconus include surgical and non-surgical methods, with a focus on restoring vision and halting disease progression. In an early stage of the disease, vision can be improved through non-surgical treatment solutions such as eyeglasses or contact lenses; however, in the advanced stage of the disease, the vision can be no longer maintained by auxiliary devices.

To halt the progression of the keratoconus, the only clinically proven effective method is corneal cross-linking (CXL) surgery. This surgery helps maintain the vision of the patient in the early stage by increasing a corneal biomechanical stiffness. The CXL surgery achieves corneal biomechanical enhancement by strengthening cross links between collagen structures in corneal stroma. The existing clinical methods typically use riboflavin as a photoinitiator and utilize ultraviolet (UV) light to induce the cross links between collagen fibers in the corneal stroma. In a healthy cornea, the cross links between collagens can be induced using copper and lysyl oxidase, but a significant decrease in a level of the lysyl oxidase in the cornea leads to insufficient cross links of the collagens. In the clinical CXL surgery involving the riboflavin and the UV light, additional covalent cross links are formed in the keratoconus through the cross links of free histidine groups on the collagens. Although the CXL surgery based on the riboflavin and UV light is the most effective non-surgical option for clinical treatment of the keratoconus, it still has significant limitations and complications. For example, the riboflavin-based CXL solution is not suitable for patients with a corneal thickness of 400 µm or less. This is because UV exposure has a negative impact on corneal endothelial cells located below the corneal stroma, resulting in irreversible endothelial toxicity and corneal edema. In addition, the clinical CXL surgery may develop postoperative complications such as permanent corneal haze, persistent corneal edema and corneal endothelial failure. The riboflavin-based CXL solution is ineffective in treating approximately 8% of the keratoconus patients, and the only possible treatment option is corneal transplantation in such cases. Despite the standard CXL surgery being modified in various aspects, none have significantly improved the efficacy of CXL treatment. Therefore, development of new cross-linking technologies for the keratoconus is a pressing key scientific issue that needs to be addressed currently and also represents an international research frontier in this field.

The corneal stroma is mainly composed of collagen fiber bundles that are arranged in parallel, which consists of finer collagen fibrils. Each spacing between the collagen fiber bundles is very uniform and extends almost in the same direction, and this arrangement ensures that light can pass straight through the cornea without being scattered, thereby guaranteeing visual clarity. In a process of assisting an collagen fiber arrangement, some bioactive proteins play a key role, for example, family members of small leucine-rich repeat proteoglycan (SLRPs) are widely distributed in the corneal stroma and have compensatory and synergistic effects therebetween, jointly regulating formation and assembly of the collagen fibers in a stromal layer, and maintaining a highly ordered arrangement of the collagen fibers. This regular arrangement is one of key factors for maintaining corneal transparency. In conclusion, the ordered arrangement of the collagen fibers in the corneal stroma is crucial for maintaining functional characteristics of the cornea. Any factor that disrupts this natural order may lead to a loss of the corneal transparency, therefore, a deep understanding of assembly and injury-repair mechanisms of the corneal stroma has important clinical significance.

Furthermore, in a treatment of repairing the cornea, how to deliver drugs across a corneal epithelial barrier to the corneal stroma is a major challenge. Corneal epithelial cells form an effective barrier that limits most molecules from penetrating. According to relevant studies, corneal epithelium consists of 6-8 layers of cells, with tight junctions formed between these cells, and diameters of paracellular pores are approximately 2 nanometers (nm), which means that only small molecules with a molecular weight of no more than 500 Daltons (Da) can pass through this tight structure. Therefore, molecules capable of penetrating intercellular spaces of the corneal epithelial should be very small. For example, small molecules such as water and oxygen can easily pass through this barrier, while larger molecules such as polypeptides or proteins have difficulty penetrating naturally. To improve an ability that the drugs penetrate the cornea, researchers have explored a variety of methods, for example, penetration enhancers are used to temporarily increase corneal permeability. Borneol, a small-molecule lipid-soluble terpenoid, has been proven to have a selective penetration-enhancing effect on hydrophilic and macromolecular drugs, it can promote penetration by altering skin stratum corneum and loosening tight junction structures between the endothelial cells. In addition, EDTA can open tight junctions between the epithelial cells, thereby promoting drug penetration through a paracellular pathway; and Tween 80 can improve cell membrane permeability by perturbing phosphatidyl chains. However, when considering any methods to enhance the corneal permeability, caution should be exercised to ensure that the natural protective function of the cornea is not impaired and unnecessary side effects are avoided. Therefore, it is very important for the researchers who wish to develop ocular drug delivery systems to understand corneal physiology and barrier properties. At the same time, when designing new drug delivery technologies, a balance between safety and efficacy also needs to be obtained.

### BRIEF SUMMARY OF THE DISCLOSURE

An objective of the present disclosure is to provide a virus-like structured nano-connector to solve the deficiencies in the existing techniques.

Another objective of the present disclosure is to provide an application of the virus-like structured nano-connector.

The technical solution of the present disclosure is as follows.

A virus-like structured nano-connector, it is based on a hepatitis B virus core protein uniprot P03146 (November 1, 1988) with a C-terminal nucleic acid-binding domain of positions 145-183 (preferably 150-183) being removed, and its spike region (between amino acid residues at positions of 78-81) and/or N-terminal are respectively inserted with a collagen-binding peptide (CBP).

In a preferred embodiment, the CBP has a sequence as shown in SEQ ID NO. 1.

In a preferred embodiment, the CBP is inserted in position 78. In another preferred embodiment, the CBP is inserted in position 79, and yet in another preferred embodiment, the CBP is inserted in position 80. In another preferred embodiment, the CBP is inserted in position 81.

In an embodiment, the CBP is inserted at N-terminal, preferably before the histidine Tag (HHHHHH).

In a preferred embodiment of the present disclosure, its amino acid sequence is as shown in SEQ ID NO. 2.

Further preferably, its nucleotide sequence is as shown in SEQ ID NO. 3.

In a preferred embodiment of the present disclosure, the virus-like structured nano-connector is based on the hepatitis B virus core protein uniprot P03146 (November 1, 1988) with the C-terminal nucleic acid-binding domain of positions 150-183 being removed.

A method of preparing a composition for promoting corneal stroma repair using the virus-like structured nano-connector.

A composition for promoting corneal stroma repair, its active ingredient comprises the virus-like structured nano-connector.

The technical solution has the following advantages.

1. The present disclosure fully uses advantages of virus-like structures, not only binding efficiency of CBP used as bionic decorin polypeptide to collagen is increased, but an efficient transcytosis process mediated by caveolae pathways is also achieved, thereby accurately delivering the present disclosure into corneal stroma.

2. In the corneal stroma, the present disclosure promotes a stable connection of collagen fibers by a non-covalent binding mode formed by simulating natural stroma, thereby achieving effects of stabilizing the corneal stroma and repairing damaged cornea.

3. A repair process of the present disclosure can be completed without light conditions, featuring higher safety and lower operational complexity, which provides new possibilities for future clinical treatments.

4. The present disclosure is constructed based on a collagen-binding polypeptide (CBP) designed by bionic decorin, it is intended to mimic interaction between the native decorin and collagen fibers, enabling efficient penetration across a corneal epithelial barrier and repairing corneal stroma via a non-photocrosslinking approach, thus overcoming limitations associated with the existing clinical therapies.

### BRIEF DESCRIPTION OF THE DRAWINGS

FIG. 1 illustrates scores of ipTM (a) and pTM (b) of compound structures of the dimer and tetramer of virus-like structure nanoemulsions (VLS NEs) predicted by an AF3 model in Embodiment 1 of the present disclosure.
FIG. 2 illustrates a difference between the compound structures of the dimer (a) and the tetramer (b) of the VLS NEs and structures of wild-type HBc VLPs evaluated in Embodiment 1 of the present disclosure, and indicators are Interface_PAE and RMSD, etc.
FIG. 3 illustrates various indicators for evaluating qualities of the compound structures of the dimer (a) and the tetramer (b) of the VLS NEs in Embodiment 1 of the present disclosure, including dG/dSASA, BUNS and packstat.
FIG. 4 illustrates molecular weights of the VLS NEs and its scaffold protein HBc149 detected by SDS-PAGE in Embodiment 1 of the present disclosure.
FIG. 5 illustrates TEM (a) analysis and DLS analysis (b) of the VLS NEs in Embodiment 1 of the present disclosure.
FIG. 6 illustrates a thermodynamic stability of the VLS NEs analyzed by a DSC (a) and DSF analyses (b) in Embodiment 1 of the present disclosure.
FIG. 7 illustrates interactions of CBP (a), dimer of the CBP (b), and the VLS NEs (c) with collagen type I analyzed by SPR in Embodiment 2 of the present disclosure.
FIG. 8 illustrates a retention situation of the VLS NEs in corneal tissues analyzed by IVIS in Embodiment 3 of the present disclosure with a scale bar of 20 µm.
FIG. 9 illustrates fluorescence microscopic analysis of sections of the corneal tissues after drug treatment in Embodiment 3 of the present disclosure with a scale bar of 20 µm.
FIG. 10 illustrates a process of penetrating the VLS NEs from corneal epithelium into the corneal tissues in Embodiment 3 of the present disclosure with a scale bar of 20 µm.
FIG. 11 illustrates a histogram of cell populations for the VLS NEs uptaken by HCECs pretreated with inhibitors at different time points in Embodiment 4 of the present disclosure.
FIG. 12 illustrates a proportion of positive cell populations (uptake percent) and mean fluorescence intensity (MFI) of the VLS NEs uptaken by the HCECs pretreated with different inhibitors (M-β-CD, EIPA and CHL), at different time points in Embodiment 4 of the present disclosure.
FIG. 13 illustrates changes in a percent of positive cell populations (a) and mean fluorescence intensity (b) and uptake inhibition relative to a positive control group (c-d) of the VLS NEs uptaken by the HCECs pretreated with the different inhibitors at different time points analyzed from a time dimension in Embodiment 5 of the present disclosure.
FIG. 14 illustrates efficiency of different inhibitors (the M-β-CD, the EIPA, the CHL, BRE.A, MON and BAF.A1) on the VLS NEs crossing HCECs barrier at different time points, studied by Transwell assay in Embodiment 5 of the present disclosure.
FIG. 15 illustrates a heatmap analysis of the efficiency of the different inhibitors (the M-β-CD, the EIPA, the CHL, the BRE.A, the MON, the BAF.A1) on the VLS NEs crossing the HCECs barrier at different time points, studied by the Transwell assay in Embodiment 5 of the present disclosure.
FIG. 16 illustrates influence of different inhibitors (the M-β-CD, the EIPA, the CHL, the BRE.A, the MON, and the BAF.A1) on the VLS NEs in the HCECs, studied by confocal experiments in Embodiment 5 of the present disclosure.
FIG. 17 illustrates results of rat corneal dissolution experiments at different time points and after drug treatment in Embodiment 6 of the present disclosure.
FIG. 18 illustrates influence of a VLS NEs treatment on various ocular tissues in mice evaluated by multiple indicators, such as direct observation, fluorescein fundus angiography, OCT and fundus imaging, in Embodiment 7 of the present disclosure.
FIG. 19 illustrates an evaluation of structural integrity of corneal endothelial cells in Embodiment 7 of the present disclosure with a scale bar of 100 µm.
FIG. 20 illustrates H&E dye images of tissue sections of heart, liver, spleen, lungs, and kidneys of mice in Embodiment 7 of the present disclosure with a scale bar of 100 µm.

### DETAILED DESCRIPTION OF THE EMBODIMENTS

The technical solution of the present disclosure will be further explained and described below using specific embodiments in conjunction with the accompanying drawings.

Female Balb/c mice and C57BL/6 mice used in the following embodiments were purchased from the Laboratory Animal Center of Shandong First Medical University. Rearing and animal experiments of mice are implemented in accordance with the requirements of the Ethics Committee of the Laboratory Animal Center of Shandong First Medical University.

In the following embodiments, preparations of common solutions are as follows:
(1) Phosphate buffered saline (PBS): 8 g of NaCl, 1.44 g of Na₂HPO₄, 0.2 g of KCl, and 0.24 g of KH₂PO₄ are accurately weighed, dissolved in 900 mL of deionized water, and fully stirred until completely dissolved. NaOH or HCl is used to adjust pH to 7.4, and deionized water is then used to dilute to a final volume of 1 L. After a preparation is complete, the PBS is autoclaved at 121 °C for 30 minutes, cooled down, and stored for later use.
(2) 1 M of Tris-HCl (pH 8.0) stock solution: 124.07 g of Tris base is accurately weighed, added into 900 mL of deionized water, and fully stirred until completely dissolved. The solution is adjusted to pH 8.0 using concentrated hydrochloric acid, finally diluted to a final volume of 1 L with deionized water, and stored for later use.
(3) 5× SDS-PAGE electrophoresis buffer stock solution: 94 g of glycine, 5 g of sodium dodecyl sulfate (SDS), and 15.1 g of Tris are respectively weighed, mixed, and dissolved in 900 mL of deionized water. After stirring until homogeneous, deionized water is used to dilute to a final volume of 1 L. The stock solution needs to be diluted 5-fold to obtain a working concentration before use.
(4) Ampicillin stock solution: ampicillin dry powder is placed at room temperature for equilibration, 5 g is then accurately weighed, dissolved in 40 mL of deionized water, and then diluted to a final volume of 50 mL. The solution should be filtered through a 0.22 µm filter membrane in a clean bench for sterilization, loaded separately into small portions, and stored at -20 °C for later use.
(5) High-salt lysis buffer solution: 58.45 g of NaCl is weighed, 20 mL of 1 M Tris-HCl (pH 8.0) stock solution, 100 mL of glycerol, and 1 mL of Triton X-100 are added, 700 mL of deionized water are added and fully stirred until all components are completely dissolved. Deionized water is finally used to dilute to a final volume of 1 L and mixed until homogeneous for a cell lysis step.
(6) Coomassie brilliant blue dye solution: 1.0 g of Coomassie brilliant blue R-250 is weighed and first dissolved in 450 mL of methanol, 100 mL of glacial acetic acid is then added, and finally dilute with 450 mL of deionized water. The dye solution is obtained by fully stirring until homogeneous and is suitable for dying protein gel.
(7) Dedye solution: 450 mL of methanol, 100 mL of glacial acetic acid, and 450 mL of deionized water are mixed in this ratio and stirred thoroughly to obtain the dedye solution for use, and excessive dye is removed from the gel, facilitating observation of protein bands.
(8) Buffer for washing endotoxinaffinity chromatography medium: 10 g of sodium deoxycholate is weighted, firstly dissolved in 800 mL of deionized water, and stirred thoroughly until all components are completely dissolved. Finally, deionized water is used to dilute to a volume of 1 L and mixed until homogeneous for washing the endotoxin affinity chromatography medium.

In the following embodiments, methods of some involved experiments are as follows:

### (1) VLS NEs uptake by HCECs is observed by a laser confocal microscope:

The VLS NEs are labeled with fluorescence FITC, and a labeling method is as follows: 2 mg of sulfo-FITC is accurately weighed, 400 µL of DMSO is added, and dissolved via a ultrasonic treatment, 10 µL is taken, added to 5 mL of the VLS NEs (1 mg/mL), reacted at room temperature for 90 minutes, loaded into a 3500 Da dialysis bag, and dialyzed in the PBS solution for 24 hours to obtain FITC-VLS NEs. Light should be avoided during the process.

The HCECs subjected to induction culture are inoculated into a 30 mm cell culture dish dedicated for laser confocal microscopy, and 5×10⁴ cells are inoculated per well and cultured overnight. The FITC-VLS NEs are added to serum-free cell culture medium, and a final concentration of the VLS NEs is 1 µg·mL⁻¹. After being cultured in a cell incubator for 2 hours, a supernatant is removed, the culture dish is washed once with the sterile PBS, a Hoechst 33342 dye solution is added and is dyed at room temperature for 15 minutes and washed the culture dish once with the sterile PBS (for subcellular localization of BMDCs, red lysosome fluorescent probes are further used to dye the cells according to the instructions). Subsequently, 500 µL of a cold 4% paraformaldehyde solution is added to fix for 10 minutes, the culture dish is washed 3 times with the sterile PBS, drops of a fluorescence quenching-resistant mounting medium are added on cell surfaces and are observed under the laser confocal microscope. When conducting operation, the light should be avoided throughout the entire process.

### (2) An endocytic pathway of the VLS NEs uptaken by the HCECs is detected using a flow cytometry:

To clarify an intracellular transport mechanism of the VLS NEs, a blocking experiment with a specific endocytic pathway inhibitor is used to analyze their dependent pathways. Corneal epithelial cells are selected and inoculated into a 24-well plate, when a cell confluency reaches 80%, the corneal epithelial cells are pretreated with M-β-CD (2 mM, caveolin-mediated endocytosis inhibitor), EIPA (100 µM, macropinocytosis inhibitor) and chloroquine (100 µM, clathrin-dependent pathway inhibitor) respectively for 1 hour (37 °C), and a fluorescent-labeled VLS NEs-drug complex is then added and is co-incubated for 2 hours. The cells are re-suspended after washing with the PBS, quantitative analysis of fluorescence intensity variations is performed via the flow cytometry, a decay rate of an uptake efficiency is obtained with a group without inhibitors as the standard reference by calculating, and contributions of various pathways to VLS NEs endocytosis are quantified through a dose-response curve.

### (3) Ability of the VLS NEs cross cell barriers of the HCECs is detected using Transwell:

A corneal epithelial barrier model is constructed based on the Transwell system, and monolayer integrity of the cells is monitored by transepithelial electrical resistance (TEER). In an experimental group, the M-β-CD (Methyl-β-cyclodextrin, caveolin-mediated endocytosis inhibitor, 2 mM), the EIPA (Ethylisopropylamiloride, macropinocytosis inhibitor, 100 µM), and the chlorquine (clathrin-dependent pathway inhibitor, 100 µM) are used to inhibit different endocytic pathways, Monensin (50 µM), brefeldin A (90 µM), and bafilomycin A1 (150 µM) are used to inhibit different transport pathways, and VLS NEs drug is added to an upper chamber after a pretreatment for 1 hour (37 °C). After 2 hours, a medium in a lower chamber is collected, quantitative analysis of drug concentrations is performed by fluorescence spectrophotometry, an inhibition rate is obtained by calculating using a transport efficiency of a control group (without inhibitors) as 100%, endocytosis and cross-organelle transport blocking data is combined to construct a transmembrane transport pathway network model of the VLS NEs, and a synergistic mechanism of subcellular organelles from which they depend is clarified.

### (4) Anti-enzymatic dissolution experiment of cornea:

Healthy female Sprague-Dawley rats with intact corneal epithelium are selected and randomly divided into 5 groups (n=5/group) after a slit-lamp microscopic examination, right eyes receive interventions respectively: ① In a control group, 200 µL of the PBS buffer (pH 7.4) is instilled; ② In a RF+UV group, 1% riboflavin solution (30 µg/mL) is topically administered followed with ultraviolet irradiation (3 mW/cm², 30 minutes); ③ In experimental groups, wild-type HBc VLPs and the VLS NEs are instilled at a same concentration respectively, all groups maintain a consistent ocular surface contact time of 30 minutes. At an end of the experiment, euthanasia is performed by cervical dislocation, and a corneal tissue is completely peeled off along a corneoscleral limbus through microsurgical techniques. Isolated corneas are placed in a 6-well plate containing 0.2% collagenase type II and oscillated at a constant temperature of 37 °C (175 rpm) until completely dissolved. During a dynamic monitoring phase, a morphology of the remaining cornea is recorded every 5 minutes through a standardized imaging system, and quantitative analysis of a decay rate of a tissue area is performed using an image J software to construct an enzymatic digestion kinetic curve.

### (5) Evaluation of ocular surface retention ability of the VLS NEs:

C57BL/6 mice (n=10) are selected, induced by anesthesia, and randomly divided into an experimental group and a control group (n=5/group). Right eyes are administered with 10 µL of VLS NEs-FITC composite nano-suspension (30 µg/mL) in the experimental group and administered with a free FITC solution (30 µg/mL) with same amount in the control group, and a corneal infiltration time of 30 minutes is both maintained. A PerkinElmer IVIS Lumina III in vivo imaging system is used and is equipped with a near-infrared filter set (excitation/emission wavelengths 750/773 nm), and dynamic fluorescence tracing on a craniofacial region is performed. Continuous monitoring is conducted for 6 hours starting from the administration, and image data is collected at 0 and 6 hours.

### (6) Isolated corneal permeability test of the VLS NEs:

Mice are euthanized by cervical dislocation, eyeballs are completely removed using corneoscleral scissors, the eyeballs are rinsed with 0.9% normal saline, and the eyeballs are placed in a mold containing OCT tissue embedding compound, and positions of the eyeballs are adjusted to be kept horizontal, and then placed in liquid nitrogen for freezing.

Completely frozen tissue embedding blocks of the eyeballs are taken out and cut into sections (10 µm) along coronal planes of the eyeballs using a cryostat, the tissue sections are attached to glass slides and observed using an inverted fluorescence microscope, fluorescent images are taken, and quantitative analysis of fluorescence intensity is performed using the Image J.

### (7) Cutting into tissue sections and dying with hematoxylin-eosin:

After an observation period is complete, section blocks of heart, liver, spleen, lung, kidney and tumor of mice in each of the groups are collected, fixed with 4% paraformaldehyde solution, then embedded with paraffin, and cut into sections, thicknesses of the sections are 7 µm, the sections are dyed with the H&E and observed under a microscope, and images are taken.

### Embodiment 1

### I. Computer-aided sequence design of the VLS NEs

A construction process of the VLS NEs in this embodiment includes the following steps:
(1) Hepatitis B virus capsid protein (HBcAg) (i.e., hepatitis B virus core protein uniprot P03146) is used as a scaffold domain to realize a self-assembly of virus-like structure nanoparticles, a C-terminal nucleic acid-binding domain (amino acid residues at positions of 150-183) of the HBcAg is removed to obtain HBc 149, thereby reducing immunogenicity and a difficulty of recombinant expression;
(2) A collagen-binding peptide (CBP, as shown in SEQ ID NO. 1: LRELHLNNN) is inserted into a spike region (between amino acid residues at positions of 78-81) of the HBc 149, and finally the VLS NEs with an amino acid sequence shown in SEQ ID NO. 2 are obtained, which can bind to collagen and assist in a formation of its complete three-dimensional structure.

SEQ ID NO. 2:

MDIDHYKEFGASVELLSFLPSDFFPSIRDLLDTASALYREALESPEHCSP
HHTALRQAILCWGELMNLATWVGSNLEDGTSGSSGSGSGGSGSGGLRELHLN
NNGSGSGGSGSGSSGSTGSRELVVGYVNVNMGLKIRQILWFHISCLTFGRETV
LEYLVSFGVWIRTPPAYRPPNAPILSTLPGSGGLRELHLNNNHHHHHH.

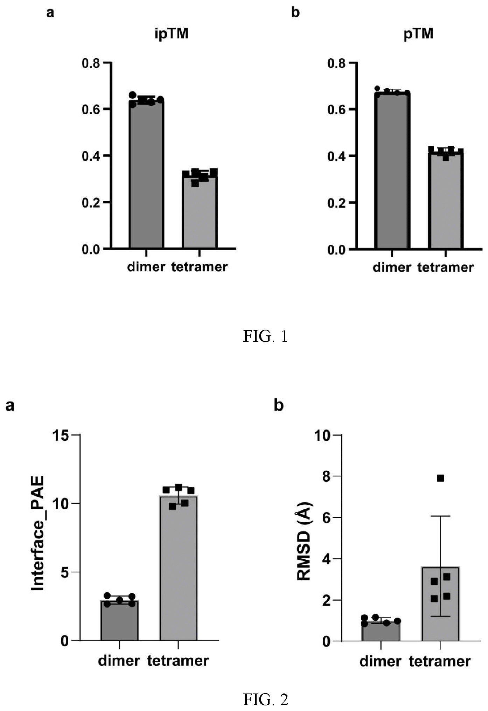

To verify that a recombinant protein sequence can maintain an integrity of a virus-like structure, in this embodiment, an AF3 model is used to perform structural simulation and evaluation analysis on its dimer and tetramer (basic structural units). Structural simulation results are shown in FIG. 1. Values of predicted template modeling (pTM) score and interface predicted template modeling (ipTM) score of simulation results of the dimer and tetramer are approximately 0.6 and approximately 0.4, respectively, and in general, the AF3 model indicates that the simulation result of the dimer is better than that of the tetramer and more reliable. Since the tetramer is a key transition state structure in a self-assembly mechanism of the HBc VLPs, 0.4 is only slightly lower than a normal standard, therefore, both of the dimer and the tetramer are analyzed in subsequent research and analysis in this embodiment.

In this embodiment, a variety of computational tools and models (such as Rosetta) are used to conduct a detailed analysis of these compound structures and interaction interfaces. The evaluation indicators include but are not limited to:
(1) RMSD (Root mean square deviation): deviation between a predicted structure and an experimental structure is evaluated;
(2) Average bidirectional minimum interface (Interface_PAE): uncertainty of the predicted structure is quantified;
(3) Binding free energy per unit area (dG/dSASA): energy density at binding interface is characterized;
(4) Number of unsatisfied bonded polar atoms (buried unsatisfied polar atoms, BUNS): potential interface unstable residues are revealed;
(5) Packing density (packing statics, packstat): a filling degree of protein interface is evaluated.

As shown in FIG. 2a, an analysis of interface predicted aligned error (the Interface_PAE) shows that a value of an alignment error of an inter-chain residue position of the dimer of the VLS NEs are all lower than 6 Å, indicating that prediction of the compound structure has high accuracy, which is consistent with an analysis result of the interface predicted template modeling (ipTM) score. It should be noted that an alignment error of an inter-chain residue position of a conformation of the tetramer increases significantly to approximately 10 Å, suggesting that compared with an oligomer structure, a prediction accuracy of the current AF3 model for a protein conformation of a multimer is relatively limited. In addition, a result of an structural similarity analysis (as shown in FIG. 2b) shows that a value of the RMSD obtained by comparing conformation of the dimer of the VLS NEs and the wild-type HBc VLPs are all less than 2 Å; and a value of the RMSD obtained by comparing the conformation of the tetramer is also generally lower than 4 Å, and no significant conformational deviation are presented. These data collectively prove that engineering modifications do not significantly change conformational characteristics of key intermediates in the self-assembly process of the VLS NEs, which supports that they have a self-assembly potential of virus-like particles comparable to that of the wild type from a perspective of structural biology.

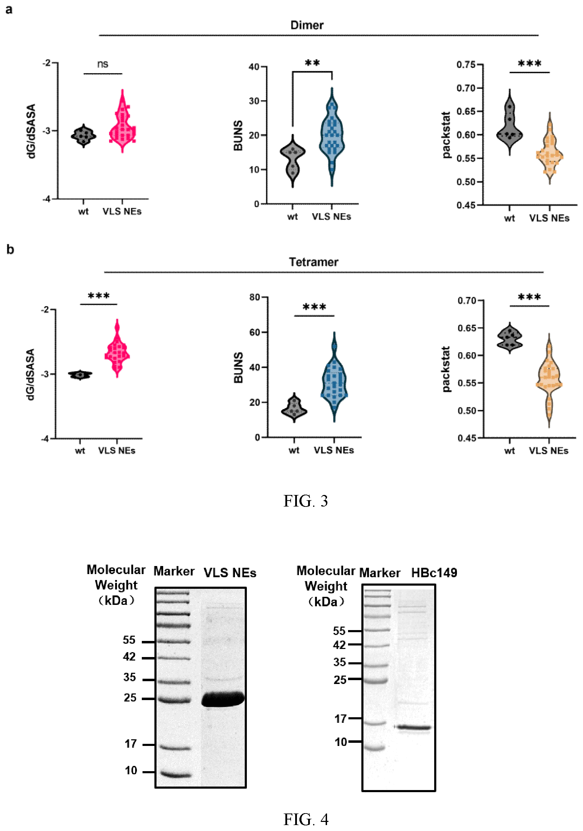

Based on the Rosetta energy analysis toolkit, this embodiment systematically evaluates an inter-chain interaction difference of the multimer of the VLS NEs and the wild-type HBc VLPs (as shown in FIG. 3). Quantitative analysis shows that a difference in the binding free energy per unit area of interfaces of the conformation of the dimer is small without significant energy disturbance; and although a difference in interaction energies in the conformation of the tetramer is statistically significant, its mean deviation is less than 1, suggesting that this energy fluctuation may have a limited influence on a self-assembly process. In addition, verification through dual indicators of the BUNS and the packstat found that the wild-type HBc VLPs shows better numerical characteristics in both parameters. Specifically, a value of the BUNS of the VLS NEs is approximately 10 higher than that of the wild type, and a score of the packstat decreases by approximately 0.15 units, this difference may be due to a redistribution of surface electrostatic potential caused by the engineering modifications. Combining the aforementioned results, it can be concluded that the VLS NEs prepared in this embodiment have a kinetic basis for assembly into complete virus-like particles, but its conformational stabilities may be weaker than that of a system of the wild-type system, which provides a clear direction for subsequent stability optimization research.

### II. Expression, purification, and characterization of the VLS NEs

In this embodiment, a pET43.1A plasmid loaded with a nucleotide sequence encoding the VLS NEs (as shown in SEQ ID NO. 3: Atggacatcgaccattataaagagttcggcgcgagcgtggaattgctgtcgttcctgccgagcgactttttcccgagcattc gtgatctgctggataccgcttctgcactctaccgcgaggccttggagagcccggaacattgcagcccgcatcacaccgca ctgcgtcaggcgattctgtgttggggtgaattaatgaacctcgctacctgggtgggttccaacttggaggacggcacgtctg gctcatctggttcaggctctggtggttcaggtagtggtggcttgcgtgaactgcacctgaacaacaacggctcaggctcagg tggcagcggtagtggtagcagcggcagtacgggtagccgtgaacttgtagttggttatgttaatgttaacatgggtctgaag atccgccaaatcttatggtttcatatcagctgcctgacgtttggccgtgagacagttttggaatacctggtctccttcggggtgt ggattcgtaccccgccggcgtatcgccctccgaatgccccaattctgtccaccctgccgggtagtggtggcttgcgtgaact gcacctgaacaacaaccaccaccaccaccaccac) is transformed into LPS-deficient *Escherichia coli* BL21 by a heat shock method. After the expression and the multi-step purification, a final product is characterized by SDS-PAGE (as shown in FIG. 4), the results shows that the VLS NEs with a high purity are obtained with all endotoxin contents being lower than 1 EU/mL. A molecular weight of the purified protein is approximately 25 kDa, which is significantly higher than that of the HBc 149 and is consistent with a theoretical value predicted using a ProtParam online tool. These results indicate that a target protein can be efficiently obtained by a purification process in this embodiment, ensuring a quality and reliability of subsequent experiments.

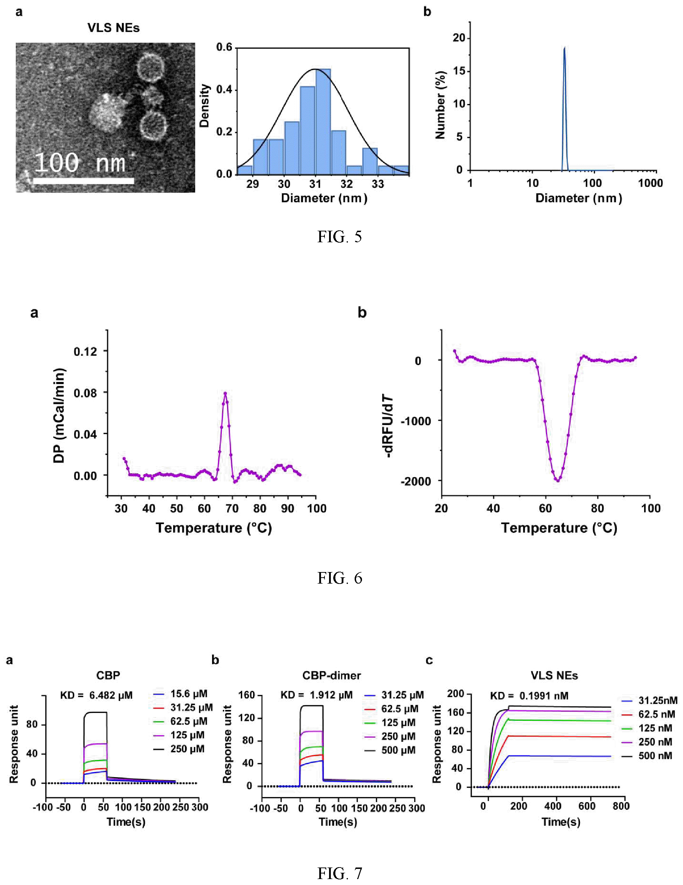

Subsequently, this embodiment uses TEM (transmission electron microscopy) and DLS (dynamic light scattering) technologies to characterize structural characteristics of the VLS NEs in detail (as shown in FIG 5). TEM images (5a) show that the VLS NEs can assemble into the complete virus-like structures, and statistical analysis shows that these particles have a uniform size with an average diameter of approximately 31 nm. In addition, DLS measurement results (FIG. 5b) further verify this finding, showing that the particle size of the VLS NEs in an aqueous solution is close to 32 nm, and a distribution the particle size is narrow. By combining these two complementary technical methods, this embodiment not only verifies morphological integrity of the VLS NEs, but an actual size in a dry state and a hydrated size under a physiological condition are also accurately quantified.

In addition, this embodiment uses DSC (differential scanning calorimetry) and DSF (differential scanning fluorimetry) to systematically evaluate thermal stability of the VLS NEs. According to an analysis of the DSC (FIG. 6a), the VLS NEs show a significant Tm of approximately 68 °C; and a value of the Tm obtained by a test of the DSF is close to 64 °C (FIG. 6b). The results of the aforementioned two methods are consistent, which indicates that the VLS NEs prepared in this embodiment have good thermal stability, which not only reflects robustness of their structures, but favorable conditions for long-term storage are also provided.

### Embodiment 2

To investigate influence displayed on surfaces of the virus-like structures on receptor-ligand affinity, this embodiment employs SPR (surface plasmon resonance) technology to determine binding constants K_{D} of the CBP, CBP-dimer (dimer) and the VLS NEs prepared in Embodiment 1 with collagen type I, respectively.

As shown in FIG. 7 and Table 1, the K_{D} of the CBP, the CBP-dimer and the VLS NEs with the collagen type I are 1.912E-6 M, 6.482E-6 M, and 1.991E-10 M, respectively. Compared with monomer and dimer forms of the CBP, the VLS NEs exhibit a binding intensity of approximately four orders of magnitude higher; and at the same time, there is no significant difference in affinity between the CBP and the CBP-dimer.

**Table 1**

| **Receptor** | **Ligand** | **K_{D} (M)** | **kₐ (1/Ms)** | **k_{d} (1/s)** |
|---|---|---|---|---|
| Collagen I | VLS NEs | 1.991E-10 | 1.129E+5 | 2.247E-5 |
| | CBP | 1.912E-6 | 7.532E+2 | 1.440E-3 |
| | CBP-dimer | 6.482E-6 | 8.821E+2 | 5.718E-3 |

Further analysis of kₐ and k_{d} reveals reasons for this significant difference include:
(1) The soluble CBP exhibits a faster dissociation rate after binding to the collagen type I. This phenomenon may be attributed to a fact that the CBP only contains binding sites without additional stable domains, resulting in a relatively large k_{d}.
(2) The CBP contains a relatively small number of binding domains, leading to a relatively small kₐ. In contrast, the VLS NEs not only provide more binding sites, but overall structural stability is also enhanced due to unique virus-like scaffold, thereby significantly improving a binding efficiency between the CBP and the collagen type I.

It should be noted that while dimerization of the CBP increases a number of potential binding sites, it does not significantly improve affinity for the collagen type I, suggesting that simple superposition of the binding sites is insufficient to enhance affinity. On the contrary, multivalent display mechanism, i.e., a presence of multiple CBP copies on each of the VLS NEs, greatly promotes efficient binding to the collagen type I. This effect is similar to an interaction mode between viruses and receptors on their host cell surfaces in nature.

In conclusion, studies in this embodiment indicate that virus-like structures used as carriers can significantly improve a binding efficiency between soluble peptides or proteins and target molecules.

### Embodiment 3

A corneal epithelial barrier is an outermost protective structure of the eyeball, composed of multiple layers of non-keratinized stratified squamous epithelial cells. These cells are rich in lipids and tightly connected, forming an effective barrier against most hydrophilic and some lipophilic drugs, which limits an efficiency of drug delivery into eyes. In addition, the normal corneal epithelial barrier is not only crucial for maintaining ocular surface health, but also plays an important role in preserving corneal transparency and visual quality.

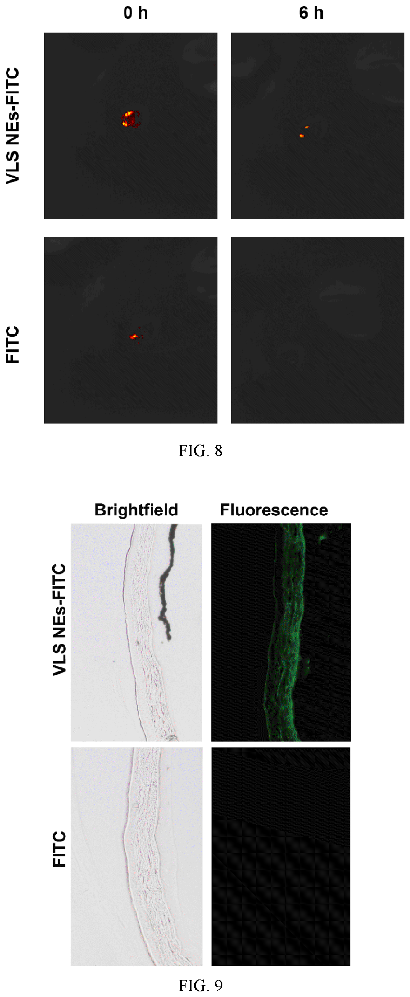

As shown in FIG. 8, in this embodiment, the FITC-fluorescently labeled VLS NEs prepared in Embodiment 1 are administered to an ocular surface of mice via ocular instillation. Fluorescence distributions on the cornea at 0 and 6 hours are analyzed using an *in vivo* imaging system (IVIS). The experimental results show that approximately 30% of the VLS NEs can achieve long-term retention in corneal tissue instead of being rapidly cleared by tear fluid, which is in sharp contrast to a result where small-molecule FITC is completely cleared quickly. These findings reveal that the VLS NEs have an ability to cross the epithelial barrier and rapidly penetrate into the corneal stroma, providing favorable conditions for subsequent treatment.

To further explore penetration and distribution situations of the VLS NEs in the corneal tissue, the excised corneal tissue is transversely sectioned in this embodiment, and cross-sections are observed using fluorescence microscopy. As shown in FIG. 9, 6 hours after ocular surface instillation, the VLS NEs can be uniformly infiltrated into and distributed in the corneal stroma, with no FITC detected. This result is consistent with previous observations, indicating that the VLS NEs have significantly superior barrier-crossing delivery performance compared to small molecules. This characteristic of the VLS NEs is of great significance for a development of new therapeutic methods for corneal diseases. A treatment of the corneal diseases often faces challenges in drug delivery because many drugs have difficulty penetrating a corneal epithelial layer, thereby limiting its therapeutic effects. As a carrier capable of penetrating the corneal epithelial layer, the VLS NEs can improve bioavailability of the drugs, enabling the drugs to reach a lesion site more effectively and thus enhancing therapeutic effects.

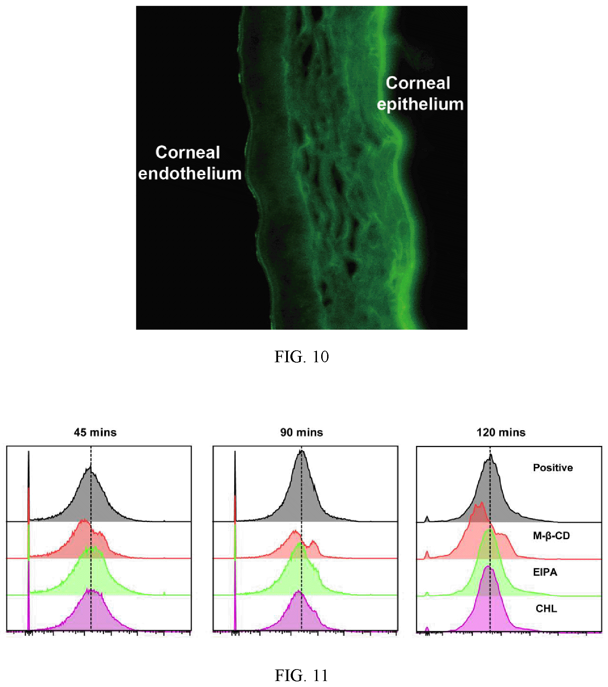

In addition, based on the aforementioned experimental results, in this embodiment, a time point of approximately 2 hours after the ocular surface instillation is selected for observation, thus detailedly analyzing a behavioral pattern of the VLS NEs crossing the barrier and diffusing in the corneal stroma. The research results are shown in FIG. 10 and indicate that a large number of the VLS NEs are endocytosed by the corneal epithelial cells and gradually transcytosed into the corneal stroma. Subsequently, the VLS NEs gradually diffuse in the stroma until being internalized by the corneal endothelial cells. From a changing tendency of fluorescence intensities, the entire penetration process is time-dependent.

### Embodiment 4

This embodiment explores a cellular transcytosis process of the VLS NEs, the process can be subdivided into two main stages: first is cellular endocytosis and second is exocytosis. For different types of endocytic pathways, this embodiment adopts specific inhibition strategies to their evaluate effects on an entry of the VLS NEs into human corneal epithelial cells (HCECs).

Specifically, this embodiment uses three different inhibitors of Methyl-β-Cyclodextrin (M-β-CD), 5-(N-Ethyl-N-Isopropyl) amiloride (EIPA), and Chloroquine (CHL). These reagents are used to block caveolae-mediated endocytosis, macropinocytosis and clathrin-dependent endocytic mechanisms, respectively. In an experimental design, Cy5.5-fluorescent-labeled VLS NEs are co-incubated with the HCECs pretreated with the aforementioned inhibitors, and multiple time points (45 minutes, 90 minutes, and 120 minutes) are set to monitor kinetic characteristics of cellular uptake of the VLS NEs.

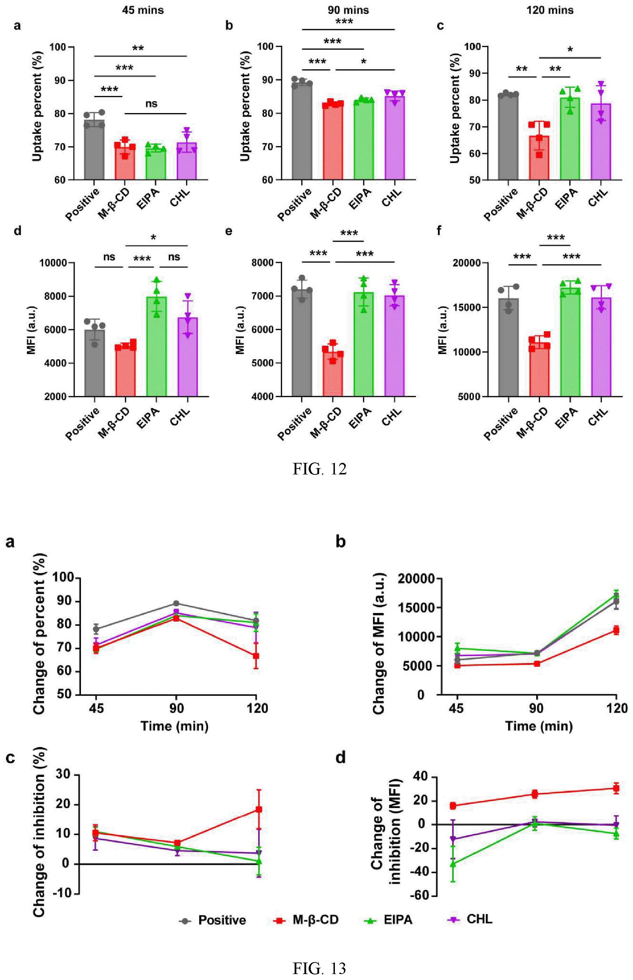

Flow cytometry analysis shows that within all tested time periods, the M-β-CD-pretreated group exhibits the most significant inhibitory effect. As shown in FIG. 11, M-β-CD-pretreated samples show an obvious population segregation phenominoun starting at 45 minutes, while no similar uptake inhibition is observed in other control groups. Furthermore, in this embodiment, quantitative analysis of a proportion of positive cell populations and its mean fluorescence intensity is performed (as shown in FIG. 12). The results show that although there are certain differences in uptake proportions among various treatment groups before 90 minutes, these differences are not significant; however, there are statistically significant differences in the mean fluorescence intensity, indicating that M-β-CD treatment significantly slows down a rate of the VLS NEs uptaken by the HCECs.

### Embodiment 5

For an in-depth understanding of time-dependent changes in inhibitory effects of different inhibitors on cellular pathways, in this embodiment, data obtained by flow cytometry is further analyzed from a time dimension (FIG. 13). This time-series analysis helps to reveal specific effects of various inhibitors on the VLS NEs uptake as time progresses. As shown in FIGS. 13a and b, after 120 minutes of co-incubation, the M-β-CD shows the most significant uptake inhibition effect compared to a positive control group and other inhibitor groups. Specifically, FIGS. 13c and d demonstrate that the M-β-CD is particularly prominent in an ability of inhibiting the VLS NEs uptaken by the HCECs, with an inhibition ratio of up to approximately 20%, while inhibitory effects of the EIPA and the CHL are relatively mild, and these effects of the two inhibitors gradually decrease to almost zero as time progresses. In terms of changes in mean fluorescence intensity, only the M-β-CD treatment group shows obvious inhibitory characteristics. Overall, the M-β-CD, as a specific caveolae inhibitor, can effectively block this process, while action mechanisms of the other two inhibitors appear to be unrelated or weakly related to this pathway. These results suggest that the VLS NEs are most likely taken up by the HCECs mainly through the caveolae-mediated endocytic pathway.

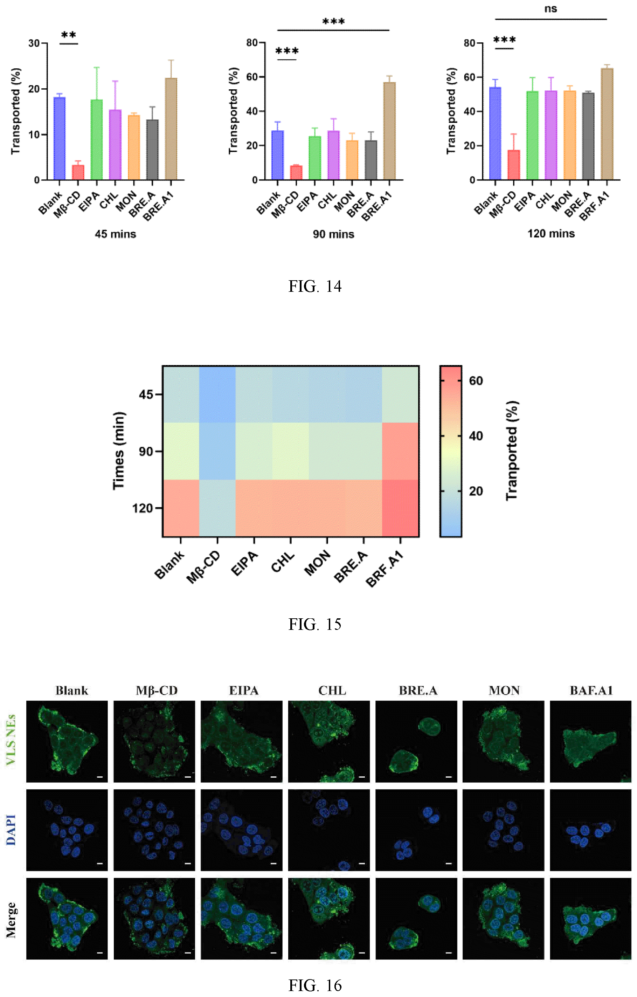

As bioactive protein nanomedicines, intracellular metabolic pathways of the VLS NEs mainly involve a lysosomal degradation system. This embodiment reveals that endocytic uptake of the VLS NEs by human coeneal epithelial cells is significantly dependent on a caveolin-mediated clathrin-independent endocytic pathway (FIG. 14). To systematically clarify its barrier-crossing transport mechanism, this embodiment uses a Transwell co-culture model combined with specific pathway inhibitors, (the M-β-CD, the EIPA, the CHL, BRE.A, MON, and BAF.A1), and quantitative analysis of functional contributions of endocytic and transcytosis pathways is performed.

The results (FIG. 14) of the Transwell experiment show that the different pathway inhibitors, such as the M-β-CD, the EIPA, the CHL, the BRE.A, the MON and the BAF.A1, have significantly different effects on an efficiency of the VLS NEs penetrating the corneal epithelial barrier. Specifically, the M-β-CD as the endocytic pathway inhibitor significantly reduces a barrier-crossing ratio of the VLS NEs, and the ratio increases as time progresses and reaches more than 30% at 120 minutes. This study is consistent with the previous endocytosis experimental results, further confirming that endocytosis is a key step for the VLS NEs to cross the corneal epithelial barrier, and the caveolae endocytic pathway is particularly important. In addition, the study also shows that when an endoplasmic reticulum-Golgi transport pathway and a Golgi-plasma membrane transport pathway are inhibited, the barrier-crossing efficiency of the VLS NEs decreases by approximately 5%, and this effect decreases as time progresses. Interestingly, after inhibiting lysosomal maturation with the BAF.A1, a transcytosis efficiency of the VLS NEs is significantly improved, especially peaking at around 90 minutes, and a growth amplitude is nearly doubled. This indicates that although a large number of the endocytosed VLS NEs are taken up and degraded by lysosomes, an increasing number of the VLS NEs are released through direct exocytosis as time progresses, thereby reducing impact from the lysosomal pathway. In summary (FIG. 15), the VLS NEs mainly rely on the caveolae-mediated endocytosis to efficiently cross the corneal epithelial barrier and then enter into the corneal stroma through various transcytosis mechanisms. It should be noted that in an initial stage, some of the VLS NEs may undergo degradation through the lysosomal pathway, this degradation phenomenon gradually decreases as an endocytic amount increases, showing unique advantages in crossing biological barriers and potential wide application prospects of the VLS NEs.

In this embodiment, this process is also observed using confocal microscopy. As shown in FIG. 16, after treatment with the M-β-CD inhibitor, uptake of the VLS NEs by the HCECs is significantly reduced, and particles are mainly concentrated on membrane surfaces of cells and have difficulty entering the cells, which is significantly different from the control group and the other pathway inhibitor treatment groups. This is consistent with results obtained in the endocytosis experiment and the Transwell experiment of this embodiment.

### Embodiment 6

This embodiment systematically evaluates the corneal repair efficacy and action mechanism of the VLS NEs by establishing a multi-species corneal injury model (such as rats, New Zealand rabbits). Photodynamic therapy (RF) and the wild-type HBc VLPs (wt HBc VLPs) are introduced as parallel controls in an experimental design to clarify the differential advantages of the VLS NEs.

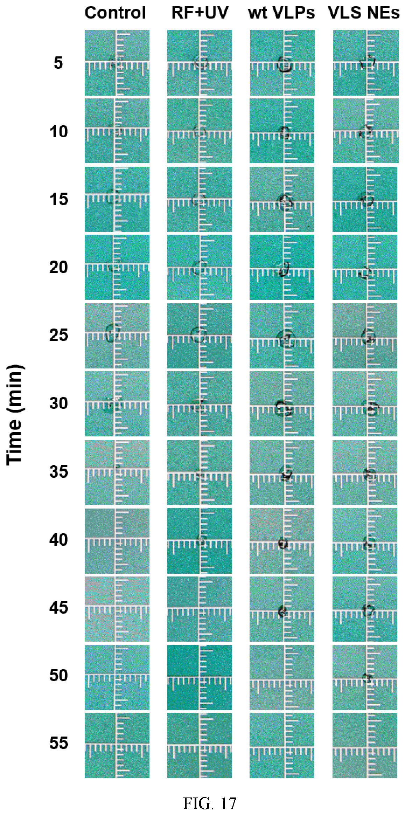

As shown in FIG. 17, in a protease-mediated corneal stromal dissolution model, the VLS NEs treatment group exhibits a significant stromal stabilization effect: a corneal tissue integrity retention rate is increased by approximately 40% compared with a control group, and a dissolution inhibition rate is increased by approximately 20% compared with the RF phototherapy group. It should be noted that complete corneal dissolution caused by insufficient penetration of RF photosensitizer after 40 minutes of treatment (a dissolution time is 10 minutes earlier than that of the VLS NEs group) is limited by a physical barrier effect of the corneal epithelial barrier. Although the wild-type HBc VLPs could delay collagen degradation through a protease competitive inhibition mechanism (a dissolution rate is reduced by 12.5%), their insufficient collagen three-dimensional cross-linking ability ed to a limited duration of therapeutic effects. The specific reasons require further research and analysis.

These results indicate that the excellent performance of the VLS NEs derived from their dual action mode:
(1) Efficient barrier penetration: the virus-like structures possess an ability to efficiently penetrate the corneal epithelial barrier and can mediate trans-epithelial transport, and the bioavailability is significantly higher than that of traditional small-molecule therapies.
(2) Based on a non-covalent interaction mechanism of natural proteins, the VLS NEs can induce cross-linking of collagen fibers in the corneal stroma under a premise of avoiding phototoxic damage, realizing reconstruction of a collagen three-dimensional network.

The aforementioned characteristics enable the VLS NEs to exhibit better efficacy and safety than first-line clinical therapies while maintaining corneal transparency, providing a new non-invasive therapeutic strategy for corneal regenerative medicine.

### Embodiment 7

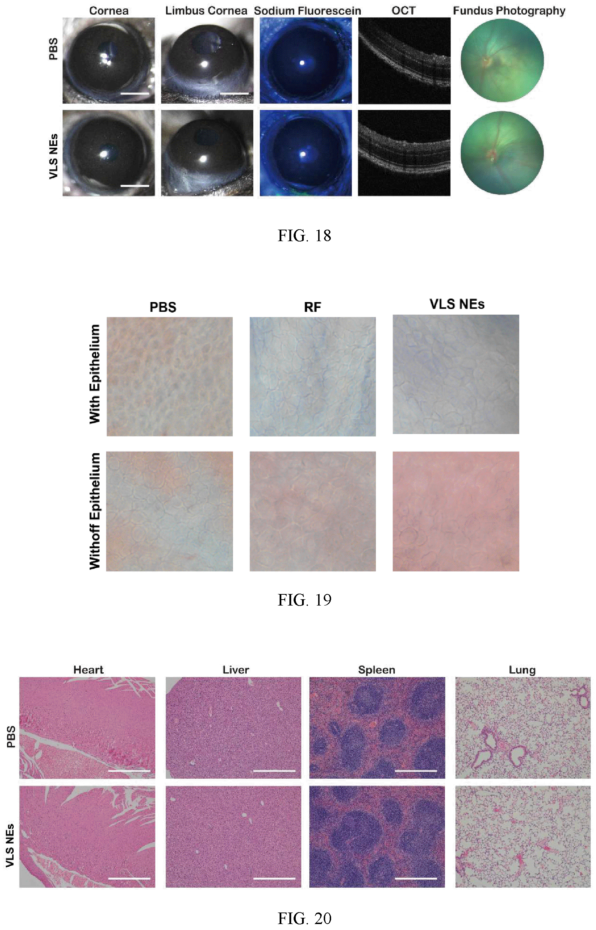

In corneal repair treatment, damaged structures may lead to significant visual loss in patients, so evaluating biosafety of the VLS NEs is particularly important. This embodiment seeks to comprehensively evaluate influence of the VLS NEs on ocular tissues through a series of rigorous experimental designs.
(1) To initially explore influence of the VLS NEs on the ocular surface, in this embodiment, an instillation treatment of the ocular surface is performed on a mouse model, and integrity and smoothness of the corneal surface are directly observed . As shown in FIG. 18, the VLS NEs do not induce obvious physical damage or rough changes on the corneal surface, indicating good biocompatibility.
(2) This embodiment focuses on a corneal limbus (limus cornea), i.e., a transition zone between the cornea and sclera, and the zone is crucial for maintaining a microenvironment for corneal stem cells. Careful examination under a microscope reveals that a structure of the corneal limbus is maintained intact without abnormalities, further confirming a safety of the VLS NEs.

To further verify whether the VLS NEs affect a retinal vascular system, in this embodiment, fundus fluorescein angiography (FFA) is performed using sodium fluorescein injection. This method can intuitively display morphology and permeability of retinal vasculature. The experimental results are shown in FIG. 18, no signs of early or subtle lesions are observed even in high-resolution images, indicating that the VLS NEs do not lead to adverse reactions in the retinal vascular system.

(3) To further explore impact of the VLS NEs on internal structures of the eyeballs, in this embodiment, non-invasive detailed detection is conducted using optical coherence tomography (OCT) and fundus imaging technology. These two technologies can provide high-resolution information on corneal structure and images of a posterior pole including retina, choroid, and optic disc. From the obtained data (FIG. 18), no abnormal changes are found in either the cornea or various layers of tissues in a posterior segment of the eyeballs, proving that the VLS NEs are not only safe for the cornea on an outer portion, but also harmless to deep ocular structures.

In conclusion, through comprehensive analysis at multiple levels, this embodiment shows that the VLS NEs have excellent biosafety and great potential in corneal repair treatment applications.

In this embodiment, to evaluate impact of the VLS NEs and the RF on ocular tissues, after completing drug administration on an ocular surface, this embodiment further collects corneal samples and examines integrity and health status of corneal endothelial cells through a microscope. The results are shown in FIG. 19, regardless of whether there is an intact corneal epithelial layer as a barrier, neither the VLS NEs nor the RF show any obvious damaging effect on corneal endothelial cells.

In a final stage of this embodiment, a safety of major organs is evaluated in mice that are subjected to ocular administration of the VLS NEs and the PBS. Specifically, at an end of the experiment, the major organs of the mice are removed for tissue sectioning, and integrity and health status of tissue structures are observed through the H&E dye method. As shown in FIG. 20, pathological examination results show that the VLS NEs exhibit good biosafety and did not cause any structural damage or abnormal changes in the major organs.

The preceding description is merely preferred embodiments of the present disclosure, so the scope of the implementation of the present disclosure is not limited thereto, thus, all equivalent variations and modifications fall into the scope of the present disclosure provided that they are obtained in accordance with the patented scope of the present disclosure and the content of the specification.

## Claims

1. A virus-like structured nano-connector, **characterized in that** the nano-connector comprises a hepatitis B virus core protein (UniProt P03146, November 1, 1988) in which the C-terminal nucleic acid-binding domain, corresponding to amino acid residues 145 - 183, has been deleted, and in which a CBP is inserted into the spike region and/or at the N-terminus, as shown in SEQ ID NO: 1, respectively.

2. The virus-like structured nano-connector according to claim 1, **characterized in that**:
its amino acid sequence is as shown in SEQ ID NO. 2.

3. The virus-like structured nano-connector according to any of claims 1 and 2, **characterized in that**: its nucleotide sequence is as shown in SEQ ID NO. 3.

4. A method of preparing a composition for promoting corneal stroma repair using the virus-like structured nano-connector according to any of claims 1-3.

5. A composition comprises the virus-like structured nano-connector according to any of claims 1-2.

6. The composition of claim 5 for use in promoting corneal stroma repair.

7. The virus-like structured nano-connector according to claim 1, **characterized in that**: the virus-like structured nano-connector is based on the hepatitis B virus core protein uniprot P03146 with the C-terminal nucleic acid-binding domain of positions 150-183 being removed.
